# FASCICULE DE BREVET EUROPEEN

(11) **EP 2 931 289 B1**
(45) Date de publication et mention de la délivrance du brevet: **17.04.2019**
(21) Numéro de dépôt: 13818279.5
(22) Date de dépôt: 10.12.2013
(51) Int. Cl.: A61K 31/722, A61K 31/7008, A61K 45/06, A61K 33/04, A61P 33/00

(54) **CHITINE OU SES DERIVES POUR LA PREVENTION ET/OU LE TRAITEMENT DE PARASITOSES**
CHITIN ODER DERIVATE DAVON ZUR PRÄVENTION UND/ODER BEHANDLUNG VON PARASITOSEN
CHITIN OR DERIVATIVES THEREOF FOR THE PREVENTION AND/OR TREATMENT OF PARASITOSES

(30) Priorité: 14.12.2012 FR 1262072
(43) Date de publication de la demande: 21.10.2015
(73) Titulaire: Lesaffre et Compagnie, 75001 Paris (FR)
(72) Inventeur: AUCLAIR, Eric, F-59710 Avelin (FR); MARDEN, Jean-Philippe, F-31670 Labege (FR); LAURENT, Fabrice, F-37210 Rochecorbon (FR); LACROIX-LAMANDE, Sonia, F-37370 Bueil en Touraine (FR)
(74) Mandataire: Cabinet Plasseraud
(86) Numéro de dépôt international: PCT/FR2013/053010
(87) Numéro de publication internationale: WO 2014/091138

(56) Documents cités:
- WO-A1-03/057233
- JP-A- 2001 081 007
- KR-B1- 100 799 652
- US-A1- 2003 185 904
- US-A1- 2012 201 916
- PETROU S ET AL: "Chitosan dipping or oregano oil treatments, singly or combined on modified atmosphere packaged chicken breast meat", INTERNATIONAL JOURNAL OF FOOD MICROBIOLOGY, ELSEVIER BV, NL, vol. 156, no. 3, 1 avril 2012 (2012-04-01) , pages 264-271, XP028488286, ISSN: 0168-1605, DOI: 10.1016/J.IJFOODMICRO.2012.04.002 [extrait le 2012-04-06]
- JULIANO CLAUDIA ET AL: "In vitro study on the anticandidal activity of Melaleuca alternifolia (tea tree) essential oil combined with chitosan", FLAVOUR AND FRAGRANCE JOURNAL, vol. 23, no. 4, juillet 2008 (2008-07), pages 227-231, XP002698734, ISSN: 0882-5734
- PRANOTO Y ET AL: "Enhancing antimicrobial activity of chitosan films by incorporating garlic oil, potassium sorbate and nisin", LWT- FOOD SCIENCE AND TECHNOLOGY, ACADEMIC PRESS, UNITED KINGDOM, vol. 38, no. 8, 1 décembre 2005 (2005-12-01), pages 859-865, XP004951627, ISSN: 0023-6438, DOI: 10.1016/J.LWT.2004.09.014
- GIATRAKOU V ET AL: "Combined Chitosan-Thyme Treatments with Modified Atmosphere Packaging on a Ready-to-Cook Poultry Product", JOURNAL OF FOOD PROTECTION, vol. 73, no. 4, avril 2010 (2010-04), pages 663-669, XP009170254, ISSN: 0362-028X

## Description

### DOMAINE TECHNIQUE

La présente invention concerne le domaine de la prévention et/ou du traitement de parasitoses, en particulier de la cryptosporidiose, chez l'homme et l'animal.

### ARRIERE-PLAN TECHNOLOGIQUE

La cryptosporidiose est une maladie liée à un protozoaire, *Cryptosporidium,* qui a des conséquences économiques négatives très importantes dans les élevages, à cause de l'augmentation de la mortalité et des retards de croissance, du coût des interventions vétérinaires et des traitements qu'elle engendre, ainsi que de l'augmentation du temps de travail pour la gestion des animaux malades.

La cryptosporidiose touche également l'homme et peut avoir des conséquences dramatiques chez les patients immunodéprimés, en particulier chez les patients atteint du VIH, ainsi que chez les nouveau-nés.

Le principal signe clinique associé à la cryptosporidiose est une diarrhée aigüe. Les autres signes cliniques associés à la diarrhée sont notamment la déshydratation, une faiblesse intense, une perte de l'appétit et des coliques. Ces différents signes cliniques peuvent entraîner la mort, notamment des jeunes animaux.

L'halofuginone est le seul médicament possédant une autorisation de mise sur le marché en France pour la prévention et/ou le traitement de la cryptosporidiose chez le veau.

Toutefois, non seulement l'efficacité de l'halofuginone est partielle, mais elle présente une certaine toxicité, un risque de développement de résistance des cryptosporidies à ce composé a été reporté (voir Silverlas et al., Preventive Veterinary Medicine, 2009, 91 : 73-84).

Par ailleurs, aucun médicament n'est enregistré pour le traitement de la cryptosporidiose chez le chevreau et l'agneau.

Chez l'homme, le nitazoxanide et la paromomycine ont été utilisés dans le traitement de la cryptosporidiose. Toutefois, à ce jour, ces molécules n'ont des autorisations de mise sur le marché que dans certains pays. Par exemple, elles n'ont pas d'autorisation de mise sur le marché en France.

Ainsi, il n'existe pas de solution réellement satisfaisante pour la prévention et/ou le traitement de la cryptosporidiose.

La chitine et le chitosan sont des composés biocompatibles, biodégradables et non toxiques, caractérisés par une forte charge négative. La chitine et le chitosan sont utilisés dans nombreuses applications très variées, allant de l'agro-alimentaire au traitement des eaux, en passant par les techniques analytiques, la cosmétique et le domaine médical (voir Shahidi et al., Trends in Food Science & Technology,1999, 10 : 37-51).

Grâce à leurs propriétés antibactérienne et antifongique, la chitine et le chitosan sont notamment utilisés comme agent de conservation et dans la fabrication de films alimentaires de protection.

Dans le domaine médical, le chitosan est par exemple utilisé comme adjuvant dans des vaccins, hémostatique, anticoagulant, antithrombogène, matrice pour la fabrications de tissus (peau, os, cartilage, foie, nerfs, vaisseaux sanguins), agent d'accélération de la cicatrisation et de traitement des brûlures, support pour le transport, l'immobilisation et l'encapsulation de molécules, telle que pour la libération contrôlée de médicaments.

Par exemple, Alvarez et al. (European Journal of Pharmaceutical Sciences, 2012, 47 : 215-227) ont testé *in vitro* l'effet de microsphères à base de chitosan et d'alcool polyvinylique qui contenaient comme médicament un complexe de furoate de diloxanide et de cyclodextrines, sur l'infection de cellules intestinales par *C. parvum.* Les auteurs indiquent que l'adhésion des microsphères aux cellules intestinales *in vitro* pourrait permettre d'inhiber l'attachement de *C*. *parvum* aux cellules intestinales et servir de système de libération du médicament. Toutefois, la capacité d'adhésion de ces microsphères aux cellules intestinales après une administration *in vivo* n'est pas démontrée.

Ainsi, il existe un réel besoin de solutions alternatives pour la prévention et/ou le traitement des parasitoses, et en particulier de la cryptosporidiose. De préférence, ces solutions alternatives n'ont pas ou peu d'effets toxiques, reposent sur des composés d'origine naturelle dont l'innocuité chez l'homme et/ou l'animal est connue, et sont simples à préparer et de mise en oeuvre.

### RESUME DE L'INVENTION

D'une façon générale, la présente invention repose sur la mise en évidence que, de manière tout à fait originale, la chitine ou des dérivés de chitine utilisés en tant que seul principe actif sont efficaces pour la prévention et/ou le traitement de parasitoses.

Par « parasitose », on entend ici une maladie liée à un protozoaire, par exemple *Cryptosporidium* dans le cas de la cryptosporidiose.

La présente invention a particulièrement pour objet la chitine ou des dérivés de chitine utilisés en tant que seul principe actif dans la prévention et/ou le traitement de la cryptosporidiose. L'invention concerne également des compositions originales associant de la chitine et / ou un dérivé de chitine en tant que principe actif avec un ou plusieurs autres composés d'origine naturelle, pour le traitement et/ou la prévention de parasitoses, et en particulier de la cryptosporidiose.

Ces compositions originales selon l'invention permettent notamment de réduire la mortalité chez les jeunes animaux et/ou la diarrhée.

De plus, ces compositions présentent l'avantage de ne contenir que des composés d'origine naturelle dont l'innocuité chez l'homme et / ou l'animal est connue aux doses conseillées d'utilisation.

Ces compositions originales n'ont ainsi pas ou peu d'effets toxiques.

Le procédé de préparation de ces compositions est simple de mise en oeuvre, ne nécessitant qu'un simple mélange des différents composés.

Ces compositions originales présentent également l'avantage de pouvoir être intégrées dans des compositions alimentaires, qu'elles soient solides ou liquides.

Un premier objet de l'invention concerne ainsi une composition comprenant :
- au moins un agent de base choisi parmi la chitine ou un dérivé de chitine, et
- un agent pour stimuler l'immunité, en tant qu'agents secondaires.

Le dérivé de chitine est choisi parmi le chitosan, la N-acétyl-glucosamine ou la glucosamine. Des agents de base préférés sont le chitosan et la N-acétyl-glucosamine.

L'agent pour stimuler l'immunité est un mélange d'écorces de levure, d'un extrait de levure, de sélénium, et d'une bactérie du genre *Bacillus..*

La composition peut en outre comprendre un agent antiparasitaire choisi parmi une huile essentielle, du charbon actif, de l'acide laurique ou leurs combinaisons.

Un agent antiparasitaire préféré comprend une huile essentielle.

Ladite composition peut être une composition alimentaire, un complément alimentaire ou une composition pharmaceutique.

Un deuxième objet de l'invention concerne de la chitine, un dérivé de chitine ou une composition telle que définie ci-dessus, pour une utilisation dans la prévention et/ou le traitement d'une parasitose, de préférence la cryptosporidiose, en particulier chez l'homme ou l'animal.

Une description plus détaillée de certains modes de réalisation préférés de l'invention est donnée ci-dessous.

### DESCRIPTION DETAILLEE

Comme indiqué précédemment, l'invention concerne des compositions à base de chitine et/ou de dérivés de chitine utilisés comme principe actif et appelés ici agents de base.

Ces compositions peuvent en outre comprendre d'autres composés, de préférence des composés d'origine naturelle, appelés ici agent secondaires.

Les agents secondaires sont choisis parmi un agent pour stimuler l'immunité et un agent antiparasitaire.

La présente invention a ainsi pour objet une composition comprenant :
- au moins un agent de base choisi parmi la chitine ou un dérivé de chitine, et
- un agent pour stimuler l'immunité tel que défini à la revendication 1.

Le dérivé de chitine est choisi parmi le chitosan, la N-acétyl-glucosamine ou la glucosamine. La chitine est un composant que l'on trouve par exemple dans l'exosquelette des arthropodes, l'endosquelette des céphalopodes et la paroi des champignons.

La chitine est un polysaccharide linéaire composé principalement de monomères de N-acétyl-glucosamine liés entre eux par des liaisons β(1-4).

La chitine est généralement obtenue par un procédé comprenant des étapes de déprotéinisation et éventuellement déminéralisation de la cuticule ou carapace des crustacés, suivies généralement d'une étape de décoloration.

L'étape de déprotéinisation est généralement une étape de traitement basique (par exemple à la soude, potasse, carbonate de sodium, carbonate de potassium ou phosphate de sodium). L'étape de déminéralisation est généralement une étape de traitement acide (par exemple avec de l'acide chlorhydrique, acide nitrique, acide sulfurique, acide acétique ou acide formique). L'étape de décoloration est par exemple effectuée par traitement avec un agent oxydant.

La chitine a un degré d'acétylation (DA) supérieur à 50%.

Le degré d'acétylation est le nombre moyen d'unités de N-acétyl-glucosamine pour 100 monomères.

On peut également utiliser le degré de désacétylation (DDA) qui est égal à 100 - DA (en pourcentage).

Par « dérivé de chitine », on entend tout composé pouvant être obtenu à partir de la chitine, en particulier par une ou plusieurs étapes d'hydrolyse, désacétylation, carboxyméthylation, succinilation ou acidification.

Un exemple de dérivé de chitine est le chitosan.

Le chitosan, appelé également chitosane, est un polysaccharide linéaire composé de monomères de glucosamine et de N-acétyl-glucosamine distribués de manière aléatoire et liés entre eux par des liaisons β(1-4).

Le chitosan a un degré d'acétylation (DA) inférieur à 50%.

Le chitosan utilisé dans les compositions selon l'invention a, de préférence, un degré d'acétylation inférieur à 40%, plus préférentiellement inférieur à 30%, plus préférentiellement encore inférieur à 20%, par exemple inférieur ou égal à 10%.

Le chitosan peut être obtenu par désacétylation chimique (par exemple par traitement dans une solution de soude concentrée), thermochimique ou enzymatique de la chitine.

Le chitosan ainsi obtenu est généralement insoluble dans les solutions aqueuses acides (notamment à un pH inférieur à 6).

Il est possible d'utiliser des méthodes bien connues de l'homme du métier pour rendre le chitosan soluble dans les solutions aqueuses acides (notamment à un pH inférieur à 6).

Par exemple, du chitosan soluble peut être obtenu par traitement à l'acide chlorhydrique. Une composition selon l'invention peut comprendre du chitosan soluble et/ou du chitosan insoluble.

Dans le cadre de la présente invention, le caractère insoluble ou soluble du chitosan est donc déterminé par mesure de sa solubilité en solution aqueuse acide, en particulier à un pH inférieur à 6.

Dans une composition préférée selon l'invention, le chitosan utilisé est un chitosan soluble.

Un autre exemple de dérivé de chitine est la N-acétyl-glucosamine, appelée également N-acétyl-D-glucosamine ou NAG.

La N-acétyl-glucosamine est par exemple obtenue par hydrolyse complète de la chitine ou du chitosan, par exemple par hydrolyse enzymatique ou acide, ou encore par N-acétylation de la glucosamine.

Encore un autre exemple de dérivé de chitine est la glucosamine, appelée également D-glucosamine.

La glucosamine est par exemple obtenue par hydrolyse complète du chitosan, telle qu'une hydrolyse enzymatique ou acide.

Les dérivés de chitine préférés pour utilisation dans les compositions selon l'invention sont le chitosan et la N-acétylglucosamine.

Une composition préférée selon l'invention comprend un seul agent de base.

Lorsque la composition ne comprend qu'un seul agent de base, l'agent de base est de préférence le chitosan ou la N-acétylglucosamine, plus préférentiellement le chitosan.

Dans un mode de réalisation avantageux, la composition selon l'invention comprend au moins deux agents de base.

Par exemple, une autre composition préférée selon l'invention peut comprendre deux agents de base.

Lorsque la composition selon l'invention comprend deux agents de base, ces deux agents de base sont de préférence le chitosan et la N-acétyl-glucosamine.

Dans un autre mode de réalisation de l'invention, la composition comprend au moins trois agents de base, par exemple trois agents de base, quatre agents de base ou plus.

Une composition préférée selon l'invention comprend, en tant qu'agents de base, du chitosan et optionnellement de la N-acétyl-glucosamine.

L'agent pour stimuler l'immunité est de préférence un composé d'origine naturelle ou une combinaison de composés d'origine naturelle.

La présente invention a ainsi pour objet une composition telle que définie ci-dessus, caractérisée en ce que l'agent pour stimuler l'immunité est un mélange d'écorces de levure, d'un extrait de levure, de sélénium, et d'un microorganisme qui est une bactérie du genre *Bacillus.*

Les écorces de levure correspondent à la fraction insoluble des levures, c'est-à-dire la paroi des levures et la membrane plasmique des levures.

Un extrait de levure correspond à la fraction soluble des levures.

La levure utilisée pour préparer les écorces de levure et/ou l'extrait de levure est de préférence choisie parmi le genre *Saccharomyces,* de préférence *Saccharomyces cerevisiae, Saccharomyces pastorianus,* ou *Saccharomyces bayanus*; le genre *Torulaspora,* de préférence *Torulaspora delbrueckii*; le genre *Lindnera,* par exemple *Lindnera jadinii*; et le genre *Kluyveromyces,* de préférence *Kluyveromyces lactis* ou *Kluyveromyces marxianus.*

Une levure préférée pour préparer les écorces de levure et/ou l'extrait de levure est *Saccharomyces cerevisiae.*

De façon classique, les écorces de levure ou l'extrait de levure sont obtenus par un procédé comprenant une étape d'autolyse des levures suivie d'une étape de séparation de la fraction soluble de la fraction insoluble, la fraction insoluble isolée correspondant aux écorces de levure et la fraction soluble correspondant à l'extrait de levure.

La fraction insoluble et/ou la fraction soluble peuvent ensuite être séchées.

Dans un mode de réalisation avantageux, les écorces de levure sont obtenues selon un procédé comprenant les étapes suivantes:
- production de levures en fermenteur pour obtenir une crème de levure,
- acidification de la crème de levure à un pH compris entre 1 et 5,
- autolyse à une température fixe ou variable comprise entre 45°C et 70°C, éventuellement en présence d'enzymes protéolytiques,
- séparation de la fraction insoluble correspondant aux écorces de levure (entre 10% et 14% de matière sèche),
- refroidissement à 4°C;
- éventuellement séchage.

Les écorces de levure peuvent se présenter sous forme liquide, sous forme sèche ou sous forme visqueuse. On considère qu'elles sont sous forme sèche lorsque leur teneur en matière sèche est d'au moins 85%, de préférence d'au moins 90%, et plus préférentiellement encore d'au moins 94% en masse. Au contraire, si leur teneur en matière sèche est inférieure à 20% en masse, on considère qu'elles sont sous forme liquide. A partir de 20% et en-dessous de 85% en masse de matière sèche, on considère que les écorces de levure sont sous forme visqueuse.

Les écorces de levure comprennent majoritairement des glucides (entre 40% et 60% de glucides en masse de matière sèche) constitués principalement de β-glucanes et de mannanes. Elles contiennent également de 10% à 30%, notamment environ 15 à 30% de matières protéiques en masse de matière sèche.

Les écorces de levure sont de préférence utilisées sous forme sèche.

Dans un mode de réalisation avantageux, l'extrait de levure est obtenu selon un procédé comprenant les étapes suivantes:
- production de levures en fermenteur pour obtenir une crème de levure,
- acidification de la crème de levure à un pH compris entre 1 et 5,
- autolyse à une température fixe ou variable comprise entre 45°C et 70°C, éventuellement en présence d'enzymes protéolytiques,
- séparation de la fraction soluble correspondant à l'extrait de levure,
- refroidissement à 4°C,
- éventuellement séchage.

L'extrait de levure peut se présenter sous forme sèche, de préférence sous forme de poudre hydrosoluble fine, sous forme liquide ou sous forme de pâte.

On considère que l'extrait de levure est sous forme sèche lorsque sa teneur en matière sèche est d'au moins 85%, de préférence d'au moins 90%, et plus préférentiellement encore d'au moins 94% en masse. Si sa teneur en matière sèche est inférieure à 70% en masse, on considère qu'il est sous forme liquide. A partir de 70% et en dessous de 85% en masse de matière sèche, on considère que l'extrait de levure est sous forme de pâte.

L'extrait de levure utilisé est de préférence sous forme sèche, plus préférentiellement sous forme de poudre hydrosoluble fine.

Un extrait de levure comprend majoritairement des matières protéiques, de préférence au moins 55 % de matières protéiques.

Le sélénium est du sélénium sous forme minérale ou sous forme organique, de préférence sous forme organique.

Le sélénium sous forme organique est par exemple sous forme de sélénométhionine, sélénocystèine, sélénoxide, S-(méthylséléno)cystéine, Se-méthylsélénocystéine, Se-adénosylhomocystéine, sélénolanthionine, sélénocystine, sélénocystathionine, γ-glutamyl-Se-méthylsélénocystéine, diméthylsélénide, diméthyldisélénide, diéthylsélénide, acide (S)-2-amino-4-(méthylsélanyl)butanoïque, acide R,S-2-hydroxy-4-méthylsélénobutanoïque ou leurs combinaisons.

Le sélénium sous forme organique est par exemple apporté sous la forme d'une levure enrichie en sélénium, de sélénium extrait totalement ou partiellement de levure ou leurs combinaisons.

Une levure enrichie en sélénium peut être obtenue par multiplication des levures en présence de sélénium.

De préférence, la levure enrichie en sélénium est une levure de *Saccharomyces cerevisiae.* La levure enrichie en sélénium est de préférence sous forme de levure inactivée, la levure étant par exemple inactivée par traitement thermique.

La levure enrichie en sélénium comprend par exemple au moins 2000 mg de sélénium par kg de matière sèche dont 97% à 99% du sélénium est sous forme organique et 63% du sélénium total est sous forme de sélénométhionine.

La levure enrichie en sélénium est utilisée de préférence sous forme de poudre.

La présente invention a ainsi particulièrement pour objet une composition telle que définie ci-dessus, caractérisée en ce que le sélénium est sous la forme d'une levure enrichie en sélénium, de sélénium extrait totalement ou partiellement de levure ou leurs combinaisons.

Dans une composition préférée selon l'invention, le sélénium est sous la forme d'une levure enrichie en sélénium.

Le microorganisme compris dans l'agent pour stimuler l'immunité selon la revendication 1 est une bactérie du genre *Bacillus.*

La composition selon l'invention peut en outre comprendre un autre agent pour stimuler l'immunité qui est un microorganisme choisi parmi une bactérie du genre *Lactobacillus, Bifidobacterium, Enterococcus, Propionibacterium, Pediococcus* ou *Lactococcus,* ou une levure du genre *Saccharomyces* ou *Kluyveromyces,* ou leurs combinaisons.

Un microorganisme du genre *Bacillus* est par exemple choisi parmi les espèces *Bacillus subtilis, Bacillus coagulans, Bacillus pumilus, Bacillus agglomerans, Bacillus clausii* ou *Bacillus cereus.*

Un microorganisme du genre *Lactobacillus* est par exemple choisi parmi les espèces *Lactobacillus johnsonii, Lactobacillus acidophilus, Lactobacillus reuteri, Lactobacillus rhamnosus, Lactobacillus casei, Lactobacillus paracasei, Lactobacillus plantarum, Lactobacillus delbrueckii, Lactobacillus brevis, Lactobacillus gasseri* ou *Lactobacillus salivarius.*

Un microorganisme du genre *Bifidobacterium* est par exemple choisi parmi les espèces *Bifidobacterium lactis, Bifidobacterium infantis, Bifidobacterium longum, Bifidobacterium bifidum, Bifidobacterium animalis, Bifidobacterium breve* ou *Bifidobacterium adolescensis.* Un microorganisme du genre *Enterococcus* est par exemple l'espèce *Enterococcusfaecium.* Un microorganisme du genre *Propionibacterium* est par exemple choisi parmi les espèces *Propionibacterium freudenreichii, Propionibacterium acidipropionici* ou *Propionibacterium jensenii.*

Un microorganisme du genre *Lactococcus* est par exemple choisi parmi les espèces *Lactococcus lactis* ou *Lactococcus thermophilus.*

Un microorganisme du genre *Saccharomyces* est par exemple choisi parmi les espèces

*Saccharomyces cerevisiae, Saccharomyces pastorianus* ou *Saccharomyces bayanus.*

Un microorganisme du genre *Kluyveromyces* est par exemple choisi parmi les espèces *Kluyveromyces lactis* ou *Kluyveromyces marxianus.*

Un microorganisme du genre *Pediococcus* est par exemple choisi parmi les espèces *Pediococcus acidilactici, Pediococcus dextrinicus* ou *Pediococcus pentosaceus.*

Dans une composition préférée selon l'invention, la bactérie du genre *Bacillus* est de l'espèce *Bacillus subtilis.*

Dans une autre composition préférée selon l'invention, le microorganisme est une bactérie du genre *Lactobacillus,* plus préférentiellement *Lactobacillus johnsonii.* Dans encore une autre composition préférée selon l'invention, le microorganisme est une combinaison d'une bactérie du genre *Bacillus* et d'une bactérie du genre *Lactobacillus,* plus préférentiellement une combinaison de *Bacillus subtilis* et de *Lactobacillus johnsonii.*

Le microorganisme peut être vivant ou désactivé.

Par « désactivé » ou encore « inactivé », on entend un microorganisme mort, c'est-à-dire dont le métabolisme est irrémédiablement arrêté.

Un microorganisme désactivé peut être obtenu par des techniques bien connues de l'homme du métier, telles qu'un traitement thermique, un traitement consistant à soumettre le microorganisme à plusieurs cycles de congélation et décongélation successives, un traitement par irradiation, un traitement par atomisation ou une combinaison de ces traitements.

De préférence, la composition selon l'invention comprend un microorganisme désactivé.

L'agent antiparasitaire est de préférence un composé d'origine naturelle ou une combinaison de composés d'origine naturelle.

L'agent antiparasitaire est par exemple choisi parmi une huile essentielle, du charbon actif, de l'acide laurique, ou leurs combinaisons.

Un agent antiparasitaire préféré consiste en une huile essentielle ou un mélange d'huiles essentielles, et optionnellement du charbon actif et/ou de l'acide laurique.

Une huile essentielle est un liquide concentré en composés aromatiques volatils d'une plante. Le terme « huile essentielle » est synonyme du terme « essence végétale ».

Les procédés d'obtention d'une huile essentielle à partir d'une plante sont bien connus de l'homme du métier.

L'huile essentielle peut par exemple être obtenue par hydrodistillation, c'est-à-dire distillation par entraînement par la vapeur d'eau, extraction aux solvants volatils, extraction par expression à froid ou extraction au CO₂ supercritique.

La présente invention a notamment pour objet une composition telle que définie ci-dessus, caractérisée en ce que l'huile essentielle est choisie parmi l'huile essentielle d'ail, l'huile essentielle de citronnelle, l'huile essentielle de cannelle, l'huile essentielle de thym, l'huile essentielle d'origan, l'huile essentielle d'arbre à thé, l'huile essentielle de citron, l'huile essentielle d'eucalyptus ou leurs combinaisons.

L'huile essentielle d'ail est riche en allicine et peut être obtenue à partir *d'Allium sativum.* L'huile essentielle de citronnelle est riche en citral et citrannal et peut être obtenue à partir de *Melissa officinalis.*

L'huile essentielle de cannelle est riche en cinnamaldéhyde et acétate de cinnamyle et peut être obtenue à partir de *Cinnamomum zeylanicum.*

L'huile essentielle de thym est riche en thymol et peut être obtenue à partir de *Thymus vulgaris.*

L'huile essentielle d'origan est riche en thymol et carvacrol et peut être obtenue à partir d'*Origanum vulgare.*

L'huile essentielle d'arbre à thé est riche en gamma-terpinène, terpinèn-4-ol et alpha-terpinéol et peut être obtenue à partir de *Melaleuca alternifolia.*

L'huile essentielle de citron est riche en limonène et peut être obtenue à partir de *Citrus limon.*

L'huile essentielle d'eucalyptus est riche en 1,8-cinéole et peut être obtenue à partir d'*Eucalyptus globulus* ou *Eucalyptus radiata.*

De préférence, l'agent antiparasitaire comprend ou consiste en un mélange d'au moins deux huiles essentielles, de préférence au moins trois huiles essentielles, plus préférentiellement encore quatre huiles essentielles.

Dans un mode de réalisation particulièrement avantageux, l'huile essentielle ou le mélange d'huiles essentielles est encapsulé dans des écorces de levure.

On parle alors d'huile essentielle encapsulée ou de mélange d'huiles essentielles encapsulé. Les procédés d'encapsulation des huiles dans des écorces de levure sont bien connus de l'homme du métier (voir par exemple le document EP0242135 ou Normand et al., Journal of Agricultural and Food Chemistry, 2005, 53 : 7532-7543).

Typiquement, l'huile essentielle encapsulée ou le mélange d'huiles essentielles encapsulé dans des écorces de levure est obtenu par une étape de mise en contact de ladite huile essentielle ou dudit mélange d'huiles essentielles à encapsuler dans une suspension comprenant les écorces de levure, suivie d'une éventuelle étape de séchage.

Les écorces de levure utilisées sont telles que définies ci-dessus.

Il s'agit préférentiellement d'écorces de levure de *Saccharomyces cerevisiae.*

La présente invention a plus particulièrement pour objet une composition telle que définie ci-dessus, caractérisée en ce que l'agent antiparasitaire comprend de l'huile essentielle d'ail, de l'huile essentielle de citronnelle, de l'huile essentielle de cannelle et de l'huile essentielle de thym.

Dans un mode de réalisation préféré, les huiles essentielles d'ail, citronnelle, cannelle et thym sont apportées sous la forme d'un mélange de ces quatre huiles essentielles.

Ledit mélange d'huiles essentielles d'ail, citronnelle, cannelle et thym est de préférence encapsulé dans des écorces de levure.

Un mélange d'huiles essentielles préféré selon l'invention comprend 3% à 7% d'huile essentielle d'ail, 7% à 13% d'huile essentielle de citronnelle, 59% à 69% d'huile essentielle de cannelle, et 21% à 32% d'huile essentielle de thym, les pourcentages étant exprimés en masse sur la masse du mélange.

Dans un autre mode de réalisation avantageux, le mélange d'huiles essentielles ci-dessus est encapsulé dans des écorces de levure de *Saccharomyces cerevisiae* et ce mélange d'huiles essentielles encapsulé comprend de 10% à 30% d'huiles essentielles en masse sur la masse totale, de préférence 20 % à 30% d'huiles essentielles en masse sur la masse totale.

Le charbon actif, appelé également charbon activé ou charbon végétal activé est une poudre noire, légère, constituée essentiellement de matière carbonée à structure poreuse, caractérisée par une très grande surface spécifique.

Le charbon actif peut être produit à partir de toute matière organique végétale riche en carbone par des procédés bien connus de l'homme du métier.

L'acide laurique, appelé également acide dodécanoïque est un acide gras à chaîne moyenne composé de 12 atomes de carbone.

Un exemple de composition selon l'invention comprend ou consiste en :
- du chitosan et/ou de la N-acétyl-glucosamine en tant qu'agents de base, de préférence du chitosan,
- un agent pour stimuler l'immunité comprenant ou consistant en :
   ∘ des écorces de levure,
   ∘ un extrait de levure,
   ∘ du sélénium, de préférence sous la forme d'une levure enrichie en sélénium, et
   ∘ un microorganisme qui est *Bacillus subtilis,* et optionnellement en plus un autre microorganisme choisi parmi *Lactobacillus johnsonii, ou Saccharomyces cerevisiae* ou leurs combinaisons,
- un agent antiparasitaire comprenant ou consistant en :
   ∘ de l'huile essentielle de thym, de l'huile essentielle de citronnelle, de l'huile essentielle de cannelle et de l'huile essentielle d'ail, de préférence sous la forme d'un mélange d'huiles essentielles encapsulé,
   ∘ optionnellement du charbon actif, et
   ∘ optionnellement de l'acide laurique.

Une composition préférée selon l'invention comprend ou consiste en :
- 2% à 15% de chitosan, de préférence à 4% à 10% de chitosan, plus préférentiellement de 6% à 10% de chitosan, et/ou 2% à 30% de N-acétyl-glucosamine, de préférence à 2% à 20% de N-acétyl-glucosamine, plus préférentiellement de 4% à 10% de N-acétyl-glucosamine, en tant qu'agents de base, (de préférence le chitosan et optionnellement la N-acétyl-glucosamine),
- un agent pour stimuler l'immunité comprenant ou consistant en :
   ∘ 25% à 85% d'écorces de levure, de préférence à 30% à 70% d'écorces de levure, plus préférentiellement de 40% à 60% d'écorces de levure,
   ∘ 1% à 15% d'un extrait de levure, de préférence à 2% à 10% d'un extrait de levure, plus préférentiellement de 2% à 5% d'un extrait de levure,
   ∘ 0,002% à 0,02% de sélénium, de préférence à 0,004% à 0,01% de sélénium, plus préférentiellement de 0,004% à 0,006% de sélénium, ou bien de 1% à 10% de levure enrichie en sélénium, de préférence de 2% à 5% de levure enrichie en sélénium, plus préférentiellement de 2% à 3% de levure enrichie en sélénium,
   ∘ de 10⁵ UFC à 10¹² UFC pour 100g de composition d'un microorganisme *Bacillus subtilis,* et optionnellement en plus un autre microorganisme choisi parmi *Lactobacillus johnsonii, Saccharomyces cerevisiae* ou leurs combinaisons, de préférence de 10⁵ UFC à 10¹¹ UFC, de préférence de 10⁵ UFC à 10¹⁰ UFC, de préférence de 10⁶ UFC à 10⁹ UFC, plus préférentiellement de 10⁶ UFC à 10⁸ UFC,
- un agent antiparasitaire comprenant ou consistant en:
   ∘ 0,4% à 20% d'huile essentielle, de préférence à 0,4% à 12%, plus préférentiellement de 1% à 6%, plus préférentiellement encore de 1,5% à 4%, l'huile essentielle étant de préférence sous la forme d'un mélange d'huiles essentielles encapsulé de thym, citronnelle, cannelle et ail, ou bien 2% à 65% d'huile essentielle encapsulée, de préférence à 2% à 40%, plus préférentiellement de 5% à 20%, plus préférentiellement encore de 8% à 12%, l'huile essentielle encapsulée étant de préférence sous la forme d'un mélange d'huiles essentielles encapsulé de thym, citronnelle, cannelle et ail,
   ∘ optionnellement 2% à 25% de charbon actif, de préférence 4% à 15%, plus préférentiellement 4% à 8%, et
   ∘ optionnellement 5% à 40% d'acide laurique, de préférence 5% à 30%, plus préférentiellement 6% à 20%.

Les pourcentages sont exprimés en g pour 100 g de composition.

Une UFC correspond à une Unité Formant Colonie.

Dans les compositions ci-dessus, les pourcentages d'écorces de levure ne comprennent pas les éventuelles écorces de levure utilisées comme agent d'encapsulation pour encapsuler les huiles essentielles.

Les compositions selon l'invention sont obtenues par une simple étape de mélange de leurs différents constituants, éventuellement suivie d'une étape de séchage.

Généralement, le mélange des différents constituants donne une composition sous forme sèche, sans nécessiter d'étape ultérieure de séchage.

Les compositions telles que définies ci-dessus sont de préférence sous forme sèche, en particulier sous forme de poudre.

Les compositions selon l'invention peuvent également être sous forme liquide.

Les compositions liquides sont généralement obtenues par mise en solution d'une composition telle que définie ci-dessus sous forme sèche.

Des compositions selon l'invention sont par exemple les compositions A, B, C et D décrites dans l'exemple 3.

La présente invention a également pour objet une composition telle que définie ci-dessus, caractérisée en ce que ladite composition est une composition alimentaire, un complément alimentaire ou une composition pharmaceutique.

La composition alimentaire peut être une composition destinée à l'alimentation humaine ou animale.

Une composition alimentaire désigne n'importe quel type d'aliment, boisson ou confiserie.

Lorsque la composition alimentaire est destinée à l'alimentation humaine, la composition alimentaire peut par exemple être une boisson, une barre de céréales, un chewing-gum, du chocolat, un produit laitier, tel qu'un produit laitier fermenté, un produit fermenté d'origine végétale.

De préférence, la composition alimentaire est destinée à l'animal.

Une composition alimentaire destinée à l'animal peut par exemple comprendre en outre un composé choisi parmi du lactosérum, de la poudre de lait, des protéines de lactosérum, du corn gluten feed, du tourteau de soja, un pré-mélange de minéraux et de vitamines, de l'huile, par exemple de l'huile de palme ou de coprah.

La composition alimentaire destinée à l'animal est par exemple de l'eau de boisson, du colostrum ou du lait, notamment un lait adapté à l'animal et son âge.

Le terme « complément alimentaire » désigne une denrée alimentaire dont le but est de compléter le régime alimentaire normal.

Un complément alimentaire constitue une source concentrée de nutriments ou d'autres substances ayant un effet nutritionnel ou physiologique, seuls ou combinés.

Un complément alimentaire est commercialisé sous forme de dose, à savoir les formes de présentation telles que gélule, pastille, comprimé, pilule et autres formes similaires, sachet de poudre, ampoule de liquide, flacon muni d'un compte-goutte et autres formes analogues de préparations liquides ou en poudres destinées à être prises en unités mesurées de faible quantité.

Une composition pharmaceutique selon l'invention est de préférence destinée à une administration par voie orale.

A titre d'exemple de composition se présentant sous une forme adaptée pour la voie orale, on peut citer un comprimé, une capsule, une gélule, un sachet, une poudre, une crème, un sirop, une pâte, un gel ou une ampoule.

La composition pharmaceutique peut être administrée en mélange avec un aliment solide ou liquide (par exemple le lait chez les jeunes animaux).

Une composition pharmaceutique peut comprendre, en plus du ou des agents de base et du ou des agents secondaires, au moins un véhicule ou excipient physiologiquement acceptable. Un véhicule ou excipient physiologiquement acceptable peut être un véhicule ou excipient approprié à l'administration chez l'homme et/ou chez l'animal.

Le véhicule ou excipient est par exemple choisi parmi ceux classiquement utilisés qui sont adaptés à la préparation de formes orales.

Une composition pharmaceutique selon la présente invention peut, en outre, comprendre au moins un principe actif pharmaceutique supplémentaire (c'est-à-dire, en plus du ou des agents de base et du ou des agents secondaires).

Par « principe actif pharmaceutique », on entend tout composé ou substance dont l'administration a un effet thérapeutique ou un effet bénéfique à la santé ou condition générale d'un patient ou d'un sujet auquel il est administré.

Ainsi, un principe actif pharmaceutique supplémentaire peut être actif contre la cryptosporidiose et/ou les signes cliniques associés tels que la diarrhée, la déshydratation, la faiblesse, la perte de l'appétit et les coliques.

Des exemples de principes actifs pharmaceutiques qui peuvent être présents dans une composition de la présente invention incluent, sans limitation, des cryptosporidiostatiques, des anti-inflammatoires, des antibiotiques, des agents antipyrétiques, des agents antiémétiques, des agents antihistaminiques, des vitamines, des agents anti-spasmodiques, etc. Dans un mode de réalisation avantageux, la composition pharmaceutique selon l'invention ne contient pas de principe actif pharmaceutique supplémentaire agissant directement contre *Cryptosporidium,* tel que par exemple un cryptosporidiostatique.

Dans un autre mode de réalisation avantageux, la composition pharmaceutique selon l'invention ne contient pas de principe actif pharmaceutique supplémentaire.

La composition pharmaceutique selon la présente invention peut être administrée en utilisant toute combinaison de dosage et de voie d'administration efficace pour obtenir l'effet thérapeutique désiré.

La quantité exacte à administrer peut varier d'un patient ou d'un animal à un autre, en fonction de l'âge, du poids, de sa condition générale, et du type de traitement préventif ou curatif.

En particulier, la présente invention a pour objet une composition telle que définie ci-dessus pour une utilisation dans la prévention et/ou le traitement d'une parasitose.

Comme indiqué précédemment, il s'agit de la première fois que l'utilisation de la chitine ou de dérivés de chitine en tant que seul principe actif est décrite dans la prévention et/ou le traitement de parasitoses, et en particulier dans la prévention et/ou le traitement de la cryptosporidiose.

Aussi, la présente invention a également pour objet la chitine et/ou un dérivé de chitine pour une utilisation dans la prévention et/ou le traitement d'une parasitose.

La présente invention a plus particulièrement pour objet de la chitine, un dérivé de chitine ou une composition telle que définie ci-dessus, pour utilisation dans la prévention et/ou le traitement d'une parasitose, caractérisée en ce que la parasitose est la cryptosporidiose.

Le microorganisme responsable de la cryptosporidiose est *Cryptosporidium,* de préférence choisi parmi les espèces *C. parvum, C. bovis, C. ryanae, C. andersoni, C. cervine, C. hominis, C. meleagridis, C. felis, C. muris, C. suis, C. baileyi* et *C*. *canis.*

Le dérivé de chitine est tel que défini ci-dessus.

Le dérivé de chitine pour une utilisation dans la prévention et/ou le traitement d'une parasitose est de préférence choisi parmi le chitosan, la N-acétyl-glucosamine ou la glucosamine.

La présente invention a particulièrement pour objet de la chitine, un dérivé de chitine ou une composition telle que définie ci-dessus, pour une utilisation dans la prévention et/ou le traitement d'une parasitose, de préférence la cryptosporidiose, chez l'homme ou l'animal.

La prévention et/ou le traitement de la cryptosporidiose chez l'animal concerne tout particulièrement les ruminants, en particulier bovins, ovins, caprins et cervidés, ainsi que le porc, la volaille, le lapin.

La prévention et/ou le traitement de la cryptosporidiose chez l'animal concerne tout particulièrement les jeunes animaux, tels que veau, chevreau et agneau.

Dans un mode de réalisation avantageux, la présente invention a pour objet de la chitine, un dérivé de chitine ou une composition telle que définie ci-dessus, pour une utilisation dans la prévention et/ou le traitement d'une parasitose, de préférence la cryptosporidiose, chez l'homme ou l'animal, l'administration de la chitine, du dérivé de chitine ou de la composition étant réalisée pendant la phase colostrale.

Lorsque la composition est administrée pendant la phase colostrale, ladite composition ne comprend de préférence pas de bactérie vivante, et plus généralement pas de microorganisme vivant.

La présente description a également pour objet une méthode de traitement préventif ou curatif de la cryptosporidiose comprenant une étape d'administration au sujet malade, homme ou animal, de chitosan, dérivé de chitine, ou d'une composition telle que définie ci-dessus.

Le dosage journalier dépend de l'homme ou l'animal, son âge et du type de traitement préventif ou curatif.

A titre d'exemple, le dosage journalier chez l'homme correspond à une administration de 10 g à 25 g de la composition selon l'invention, de préférence de 15 g à 20 g de la composition. La dose journalière peut être administrée en une, deux ou trois fois.

Egalement à titre d'exemple, le dosage journalier chez l'animal peut être de :
- 2 g à 10 g de la composition selon l'invention par jour, pendant 7 jours consécutifs pour un animal d'un poids vif de 3 kg à 5 kg,
- 5 g à 15 g de la composition selon l'invention par jour, pendant 7 jours consécutifs pour un animal d'un poids vif de 30 kg à 50 kg,
- 10 g à 20 g de la composition selon l'invention par jour, pendant 7 jours consécutifs pour un animal d'un poids vif supérieur à 50 kg.

Aux doses testées, il n'a pas été observé d'effet secondaire des compositions selon l'invention sur les animaux.

D'autres caractéristiques et avantages de l'invention apparaîtront encore mieux à la lecture des exemples de réalisation suivants qui illustrent l'invention sans la limiter, et pour la compréhension desquels on se reportera aux dessins annexés.

### EXEMPLE 1 : INHIBITION DE LA MULTIPLICATION DE C. PARVUM IN VITRO

Expériences réalisées en collaboration avec l'équipe « parasites transmis par les aliments » de l'Unité Mixte de Recherche Biologie moléculaire et Immunologie Parasitaire et fongique (BIPAR), à l'Ecole Nationale Vétérinaire d'Alfort.

### Matériel et Méthodes

### (i) Lignées cellulaires

Deux modèles cellulaires ont été testés : les cellules HCT-8 (human ileocecal adenocarcinoma cell) et les cellules Caco-2 (human colonic adenocarcinoma cell).

### (ii) Composés testés : chitosan, NAG, Paromomycine

Le chitosan soluble est du chlorhydrate de chitosan avec un degré de désacétylation supérieur ou égal à 90% et un degré de viscosité de 5,5 mPas (viscosité dynamique mesurée à 20°C dans une solution d'eau distillée à 0,5%) (Kraeber & Co GMBH, Allemagne).

La NAG (N-Acétyl-glucosamine) provient de Kraeber & Co GMBH, Allemagne.

La Paromomycine (Paromomycine sulphate 100%, Antibioticos SPA, Italie) est un médicament antimicrobien utilisé dans le traitement de la cryptosporidiose qui permet la réduction de l'excrétion d'oocystes.

### (iii) C. parvum

La souche de *Cryptosporidium parvum* Iowa (Waterborne inc., New Orleans, LA, USA) est utilisée pour les essais.

### (iv) Infection des cellules

L'infection est effectuée sur des cellules à 70%-80% de confluence cultivées en monocouche sur des lamelles recouvertes de collagène dans des plaques de 24 puits.

Le surnageant des cellules en monocouche est remplacé par 3 ml de milieu de culture auxquels on ajoute 1.10⁴ oocystes de *C. parvum.* Après 3 heures d'incubation dans un thermostat humide à 37°C et 5% CO₂, le surnageant est remplacé par 3 ml de milieu de culture et mis en incubation pendant 48h dans un thermostat humide à 37°C, 5% CO₂ et 80% d'humidité.

### (iv) Test de pré-incubation

Dans chaque puits d'une plaque sont mis en contact 1.10⁴ oocystes avec 500 µg/ml de paromomycine, chitosan ou NAG.

Les plaques sont mises en incubation pendant 24h dans un thermostat humide à 37°C et 5% CO₂.

L'infection des cellules est alors réalisée comme indiqué ci-dessus.

### (v) Test direct

Le milieu de culture des cellules infectées est remplacé avec 1 ml de milieu de culture dans lequel on ajoute ensuite le composé à tester, à une concentration finale de 500µg / ml.

Les plaques sont mises en incubation pendant 48h dans un thermostat humide à 37°C et 5% CO₂.

### (vi) Coloration et comptage des oocystes intracellulaires

Les oocystes intracellulaires sont colorés après fixation des cellules au méthanol. Le marquage est effectué en utilisant un anticorps polyclonal conjugué spécifique (Sporo-Glo 20X Waterborne inc., New Orleans, LA, USA). Les lamelles sont ensuite montées sur des lames. Le comptage des oocystes intracellulaires est effectué au microscope à fluorescence. Sur chaque lame, 6 x 50 champs choisis de manière aléatoire sur toute la surface de lame sont comptés.

### (vii) Analyse statistique

Les résultats obtenus sont une moyenne de triplicat. Les données sont analysées par ANOVA. Une valeur p (p-value) inférieure ou égale à 0,05 est considérée comme significative.

### Résultats

### (i) Cytotoxicité

Des essais préliminaires ont permis de déterminer les doses optimales tolérées par les cellules HCT-8 et Caco-2 et ont conduit à choisir la dose de 500 µg/ml pour le test de chacun des composés.

### (ii) Effet de la pré-incubation des composés testés avec les oocystes de C. parvum avant l'infection

Les composés testés sont mis en incubation pendant 24h avec des oocystes de *C. parvum* avant l'infection *in vitro* à une dose de 500 µg/ml.

Les résultats obtenus sont présentés dans le tableau 1 pour les cellules HCT-8 et le tableau 2 pour les cellules Caco-2.

La Paramomycine est capable d'inhiber le développement du parasite de manière significative à la fois sur les cellules HCT-8 et Caco-2 (p<0,001) : la Paromomycine réduit de 99,5% le développement du parasite sur les cellules HCT-8 et de 97,1% sur les cellules Caco-2.

Il en va de même pour le chitosan et NAG.

Le chitosan soluble réduit respectivement de 95,7% le développement du parasite sur les cellules HCT-8 et de 97,6% sur les cellules Caco-2 (p<0,001).

La NAG réduit respectivement de 99,0% le développement du parasite sur les cellules HCT-8 et de 97,4% sur les cellules Caco-2 (p<0,001).

**Tableau 1**

| **Essai** | **Cellules HCT-8** | **Nombre de sporozoïtes** | **Moyenne** | **Ecart-type** | **% de sporozoïtes viables** |
|---|---|---|---|---|---|
| 1 | T+ | 1040 | 1046 | 10,68 | 100 |
| 2 | T+ | 1037 | | | |
| 3 | T+ | 1061 | | | |
| 4 | Paromomycine | 3 | 6 | 2,49 | 0,53 |
| 5 | Paromomycine | 9 | | | |
| 6 | Paromomycine | 5 | | | |
| 7 | NAG | 12 | 11 | 2,49 | 0,99 |
| 8 | NAG | 14 | | | |
| 9 | NAG | 7 | | | |
| 10 | chitosan | 47 | 47 | 6,24 | 4,3 |
| 11 | chitosan | 52 | | | |
| 12 | chitosan | 37 | | | |
| 13 | T- | 0 | 0 | 0 | 0 |

| | | | | | |
|---|---|---|---|---|---|
| T+ : cellules infectées / T- : cellules non infectées | | | | | |

**Tableau 2**

| **Essai** | **Cellules Caco-2** | **Nombre de sporozoïtes** | **Moyenne** | **Ecart-type** | **% de sporozoïtes viables** |
|---|---|---|---|---|---|
| 1 | T+ | 844 | 1146 | 298,7 | 100 |
| 2 | T+ | 1553 | | | |
| 3 | T+ | 1042 | | | |
| 4 | Paromomycine | 23 | 33 | 12,97 | 2,87 |
| 5 | Paromomycine | 51 | | | |
| 6 | Paromomycine | 24 | | | |
| 7 | NAG | 21 | 30 | 9,0 | 2,61 |
| 8 | NAG | 39 | | | |
| 9 | NAG | 31 | | | |
| 10 | chitosan | 19 | 28 | 10,8 | 2,44 |
| 11 | chitosan | 40 | | | |
| 12 | chitosan | 25 | | | |
| 13 | T- | 0 | 0 | 0 | 0 |

| | | | | | |
|---|---|---|---|---|---|
| T+ : cellules infectées / T- : cellules non infectées | | | | | |

### (iii) Effet des composés testés sur le développement de C. parvum lors d'un ajout direct sur les cellules infectées

Les différents composés sont testés à une dose de 500 µg/ml.

Les résultats sont présentés dans les tableaux 3 (cellules HCT-8) et 4 (cellules Caco-2).

**Tableau 3**

| **Essai** | **Cellules HCT-8** | **Nombre de sporozoïtes** | **Moyenne** | **Ecart-type** | **% de sporozoïtes viables** |
|---|---|---|---|---|---|
| 1 | T+ | 393 | 345 | 37,6 | 100 |
| 2 | T+ | 342 | | | |
| 3 | T+ | 301 | | | |
| 4 | Paromomycine | 178 | 191 | 12,6 | 55,4 |
| 5 | Paromomycine | 187 | | | |
| 6 | Paromomycine | 208 | | | |
| 7 | NAG | 172 | 134 | 29,9 | 11,6 |
| 8 | NAG | 131 | | | |
| 9 | NAG | 99 | | | |
| 10 | chitosan | 90 | 73 | 11,8 | 6,4 |
| 11 | chitosan | 66 | | | |
| 12 | chitosan | 64 | | | |
| 13 | T- | 0 | 0 | 0 | 0 |

| | | | | | |
|---|---|---|---|---|---|
| T+ : cellules infectées / T- : cellules non infectées | | | | | |

La Paromomycine, NAG et le chitosan induisent une réduction significative du développement du parasite dans les deux lignées cellulaires (p<0,005) :
- respectivement de 44,6%, 88,4% et 93,6% sur les cellules HCT-8, et
- respectivement de 33,6%, 35,2% et 68% sur les cellules Caco-2.

Ainsi, NAG et le chitosan sont plus efficaces que la Paromomycine sur les cellules HCT-8 et le chitosan est le plus efficace sur les cellules Caco-2.

**Tableau 4**

| **Essai** | **Cellules Caco-2** | **Nombre de sporozoïtes** | **Moyenne** | **Ecart-type** | **% de sporozoïtes viables** |
|---|---|---|---|---|---|
| 1 | T+ | 774 | 881 | 79,0 | 100 |
| 2 | T+ | 908 | | | |
| 3 | T+ | 962 | | | |
| 4 | Paromomycine | 318 | 592 | 193,4 | 66,4 |
| 5 | Paromomycine | 782 | | | |
| 6 | Paromomycine | 675 | | | |
| 7 | NAG | 656 | 571 | 85,5 | 64,8 |
| 8 | NAG | 485 | | | |
| 9 | NAG | 337 | | | |
| 10 | chitosan | 372 | 282 | 70,8 | 32,0 |
| 11 | chitosan | 199 | | | |
| 12 | chitosan | 276 | | | |
| 13 | T- | 0 | 0 | 0 | 0 |

| | | | | | |
|---|---|---|---|---|---|
| T+ : cellules infectées / T- : cellules non infectées | | | | | |

### EXEMPLE 2 : ADHESION OU REPLICATION DE C. PARVUM DANS LES ENTEROCYTES IN VITRO

### Matériel et Méthodes

### (i) Lignées cellulaires testées et oocystes

Les oocystes de *C. parvum* utilisés ont été isolés de fèces (selles) de veaux infectés expérimentalement (par l'INRA de Tours).

Les essais sont réalisés sur des cellules de la lignée murine CMT-93 (sans mycoplasme) cultivées en milieu complet comprenant 10% de SVF (*Sérum de Veau Foetal*).

### (ii) Composés testés

Le chitosan insoluble est du Poly-(N-déacétyl-D-glucosamine) avec une matière sèche de 6,43% et un degré de désacétylation de 95,5% (Fédéral Laboratories Chemical Corp, NY, USA).

Le chitosan soluble est du chlorhydrate de chitosan avec un degré de désacétylation supérieur ou égal à 90% et un degré de viscosité de 5,5 mPas (viscosité dynamique mesurée à 20°C dans une solution d'eau distillée à 0,5%) (Kraeber & Co GMBH, Allemagne).

La NAG (N-Acétyl-glucosamine) provient de Kraeber & Co GMBH, Allemagne.

Sont également testés :
- des écorces de levure (Safmannan, Lesaffre),
- une levure enrichie en sélénium (Selsaf, Lesaffre),
- du charbon actif,
- de l'huile essentielle d'ail,
- un mélange d'huiles essentielles (comprenant 6% d'huile essentielle d'ail, 10 à 13% d'huile essentielle de citronnelle, 59 à 72% d'huile essentielle de cannelle, et 23 à 32% d'huile essentielle de thym),
- une levure de *Saccharomyces cerevisiae* vivante (Actisaf, Lesaffre).

L'interféron y à 10 ng/ml est utilisé comme témoin positif. En effet, il est décrit une inhibition partielle de la multiplication de *C. parvum* dans les entérocytes sous l'action de cette cytokine.

### (iii) Test de l'effet des composés sur le développement de C. parvum

Les cellules de la lignée CMT-93 sont ensemencées à raison de 3,6.10⁵ cellules par puits.

Les composés à tester sont ajoutés au bout de 8 h. Après 16h d'incubation, une infection est ensuite réalisée avec des oocystes de *C. parvum* (ratio 5 oocystes / cellule) en présence des composés testés, aux doses sélectionnées qui n'affectent pas la viabilité cellulaire de plus de 15%.

Après 24h, les cellules sont lysées à la trypsine et marquées avec une lectine fluorescente (VVL-FITC), afin de pouvoir repérer les cellules infectées au microscope à fluorescence. Le nombre de cellules infectées est repéré manuellement. Cette technique est plus laborieuse, mais s'est révélée plus précise qu'un dénombrement des cellules parasitées par cytométrie.

### Résultats

Le chitosan soluble et la NAG testées à 18 µg/ml réduisent de 31% et 43% respectivement la multiplication parasitaire dans les cellules de la lignée CMT-93 par rapport au témoin non traité. Ces réductions sont similaires à celle observée pour le témoin positif, l'interféron y.

Le chitosan insoluble testé à 18 µg/ml permet une réduction moins importante de la multiplication parasitaire, avec environ 40% de cellules infectées contre 50% de cellules infectées pour le témoin non traité.

Le charbon actif à la dose de 18 µg/ml réduit de 36% la multiplication parasitaire par rapport au témoin non traité. A cette dose, l'effet du charbon reste similaire à l'interféron y.

Le mélange d'huiles essentielles (comprenant 6% d'huile essentielle d'ail, 10 à 13% d'huile essentielle de citronnelle, 59 à 72% d'huile essentielle de cannelle, et 23 à 32% d'huile essentielle de thym) à la dose de 3,3 µg/ml ou 30 µg/ml permet également une réduction de 30% de la multiplication parasitaire par rapport au témoin non traité et similaire à celle observée pour l'interféron y.

Aucun effet n'est observé avec les autres composés aux doses testées sur la multiplication parasitaire.

### EXEMPLE 3 : REPONSE IMMUNE DES CELLULES EPITHELIALES ET DES CELLULES DENDRITIQUES

### (i) Lignées cellulaires testées et oocystes

Les oocystes de *C. parvum* utilisés ont été isolés de fèces (selles) de veaux infectés expérimentalement (par l'INRA de Tours).

Les essais sont réalisés sur des cellules de la lignée murine CMT-93 (sans mycoplasme) cultivées en milieu complet comprenant 10% de SVF (*Sérum de Veau Foetal*) ou sur des cellules dendritiques dérivées de moelle osseuse (immature) en présence de GM-CSF (*Granulocyte Macrophage Colony Stimulating Factor*) ajouté régulièrement dans la culture pendant 9 jours.

### (ii) Composés testés

Les produits testés sur les cellules épithéliales sont :
- NAG (N-Acétyl-glucosamine) (Kraeber & Co GMBH, Allemagne).
- des écorces de levure (Safmannan, Lesaffre),
- une levure de *Saccharomyces cerevisiae* vivante (Actisaf, Lesaffre),
- une souche de *Lactobacillus johnsonii* inactivée

Les produits testés sur les cellules dendritiques sont :
- des écorces de levure (Safmannan, Lesaffre),
- une levure de *Saccharomyces cerevisiae* vivante (Actisaf, Lesaffre),
- une souche de *Bacillus subtilis,*
- une souche de *Lactobacillus johnsonii* vivant
- une souche de *Lactobacillus johnsonii* inactivée
- une souche d'*E*. *coli.*

### (iii) Test de la réponse immune des cellules épithéliales et dendritiques

### - Cellules épithéliales

La production de chimiokine par l'épithélium ou d'autres cellules intestinales est importante pour le recrutement des cellules inflammatoires nécessaires à la protection contre la cryptosporidiose chez les nouveau-nés. La production de chimiokine par les cellules épithéliales est mesurée par RT-PCR quantitative (quantification par incorporation de Sybr green) après incubation des cellules CMT-93 avec les différents composés pendant 24h.

### - Cellules dendritiques

Les essais sont réalisés sur des cellules dendritiques dérivées de moelle osseuse (immature) en présence de GM-CSF (*Granulocyte Macrophage Colony Stimulating Factor*) ajouté régulièrement dans la culture pendant 9 jours.

La production de la cytokine IL-12p40 est mesurée par ELISA 24h après adition des produits à différentes dilutions. Un antibiotique est ajouté 4h après l'inoculation des produits vivants.

### Résultats

Pour les cellules épithéliales, des chimiokines (CXCL2, CCL3, CCL20, CXCL10, CCL2) capables d'attirer les phagocytes mononucléés sont produites par les cellules épithéliales *in vitro* en réponse aux composés testés, toutefois en quantité moindre que par l'infection parasitaire. Le produit NAG se montre souvent la plus efficace pour CXCL10 et CCL20, puis les écorces de levure, et la levure vivante (Actisaf) pour CCL3.

Pour les cellules dendritiques, les écorces de levure, la souche de *Bacillus subtilis,* les lactobacilles, *E. coli,* et la levure vivante induisent tous de très fortes productions d'IL-12p40. Cette expérience a été reprise sur des macrophages différenciés *in vitro* (en présence de M-CSF pendant 6 jours) et une très forte réponse est obtenue pour les lactobacilles ainsi que la levure vivante.

### EXEMPLE 4 : TRAITEMENT DE LA CRYPTOSPORIDIOSE INDUITE CHEZ LE CHEVREAU

### Matériel et Méthodes

Modèle chevreau

### (i) Compositions testées

| **Composés** | **1^{er} essai** | **2^{ème} essai** | | |
|---|---|---|---|---|
| | **Composition A** 2,5 g, 5g ou 10 g / chevreau /repas * | **Composition B** 5g / chevreau / repas * | **Composition C** 7,5g / chevreau / repas * | **Composition D** 5g / chevreau / repas * |
| Ecorces de levure | 70% | 76% | 51% | 38% |
| Extrait de levure | 3% | 3% | 2% | 2% |
| Levure enrichie en sélénium | 3% | 3% | 2% | 2% |
| Levure vivante | 0% | 0% | 33% | 50% |
| NAG | 6% | 0% | 0% | 0% |
| Chitosan | 6% (soluble) | 8% (insoluble) | 5% (insoluble) | 4% (insoluble) |
| Charbon actif | 6% | 0% | 0% | 0% |
| Mélange d'huiles essentielles | 6% | 10% | 7% | 5% |

| | | | | |
|---|---|---|---|---|
| * *Les chevreaux sont nourris deux fois par jour.* | | | | |

Les écorces de levure est le produit Safmannan (Lesaffre).

L'extrait de levure est le produit EXL2020 (Biospringer).

La levure enrichie en sélénium est le produit Selsaf® (Lesaffre).

La levure vivante correspond à la souche déposée le 2 décembre 2010 à la Collection Nationale de Cultures de Microorganismes (CNCM, Institut Pasteur, 25 Rue du Docteur Roux, F-75724 PARIS Cedex 15) sous le numéro I-4407 (Actisaf, Lesaffre, à 9.10⁹ UFC/g). Le mélange d'huiles essentielles comprend 6% d'huile essentielle d'ail, 10 à 13% d'huile essentielle de citronnelle, 59 à 72% d'huile essentielle de cannelle, et 23 à 32% d'huile essentielle de thym.

La NAG provient de Kraeber & Co GMBH, Allemagne.

Le chitosan soluble est du chlorhydrate de chitosan avec un degré de désacétylation supérieur ou égal à 90% et un degré de viscosité de 5,5 mPas (viscosité dynamique mesurée à 20°C dans une solution d'eau distillée à 0,5%) (Kraeber & Co GMBH, Allemagne).

Le chitosan insoluble est un chitosan avec un degré de désacétylation supérieur à 90% (Federalabs, Etats-Unis).

Les compositions A, B, C et D sont sous forme de poudre.

### (ii) 1^{er} essai

Les chevreaux sont répartis en 4 groupes de 11 chevreaux âgés de 2 à 4 jours :
- Groupe 1 : témoin infecté ne recevant pas de traitement
- Groupe 2 : 2,5 g de la composition A par chevreau, 2 fois par jour
- Groupe 3 : 5 g de la composition A par chevreau, 2 fois par jour
- Groupe 4 : 10 g de la composition A par chevreau, 2 fois par jour

A noter que les chevreaux ont tous été traités par métaphylaxie avec de la marbofloxacine en injection sous-cutanée à la dose de 0,1 ml/kg une fois par jour, en raison de lésions de pneumonie détectées chez des chevreaux morts avant la répartition dans les 4 groupes. Ce traitement n'interfère pas avec l'essai clinique puisque la marbofloxacine n'est pas efficace contre la cryptosporidiose.

Les chevreaux sont infestés expérimentalement par voie orale à l'aide d'une seringue à une dose de 10⁶ oocystes de *Cryptosporidium parvum* (Waterborne Inc, New Orleans, USA) remis en suspension dans de l'eau.

La distribution de la composition A se fait après mise en suspension dans 100 ml de lait (aliment complet d'allaitement Optiprim du groupe Sofivo). La mise en solution de la composition est faite extemporanée, juste avant la distribution.

La distribution du mélange est faite de manière individuelle, au biberon par souci de commodité, chaque chevreau recevant 100 ml de mélange.

La distribution du traitement a lieu matin et soir pendant 14 jours et l'administration a commencé une demi-journée avant l'infestation expérimentale. La distribution du traitement a lieu avant le repas pour garantir une meilleure prise du traitement.

### (iiii) 2^{ème} essai

Les chevreaux sont répartis en 4 groupes de 11 chevreaux âgés de 2 à 4 jours :
- Groupe 1 : témoin infecté ne recevant pas de traitement
- Groupe 2 : 5 g de la composition B par chevreau, 2 fois par jour
- Groupe 3 : 7,5 g de la composition C par chevreau, 2 fois par jour
- Groupe 4 : 5 g de la composition D par chevreau, 2 fois par jour

Les chevreaux sont infestés expérimentalement par voie orale à l'aide d'une seringue à une dose de 10⁶ oocystes de *Cryptosporidium parvum* (Waterborne Inc, New Orleans, USA) remis en suspension dans de l'eau.

La distribution des compositions B, C et D se fait après mise en suspension dans 100 ml de lait (aliment complet d'allaitement Optiprim du groupe Sofivo). La mise en solution de la composition est faite extemporanée, juste avant la distribution.

La distribution du mélange est faite de manière individuelle, au biberon par souci de commodité, chaque chevreau recevant 100 ml de mélange.

La distribution du traitement a lieu matin et soir pendant 14 jours et l'administration a commencé une demi-journée avant l'infestation expérimentale. La distribution du traitement a lieu avant le repas pour garantir une meilleure prise du traitement.

### (iii) Mesures et collecte des données

Des matières fécales sont prélevées quotidiennement sur chaque chevreau afin de mesurer l'excrétion individuelle par la méthode semi-quantitative de Heine. Les prélèvements sont effectués dans le rectum.

Une note d'excrétion est attribuée pour chaque chevreau :
- 0 = absence d'oocyste
- 1 = moins de 1 oocyste
- 2 = 1 à 10 oocystes
- 3 = 11 à 20 oocystes
- 4 = 21 à 30 oocystes
- 5 = 31 à 40 oocystes.

Une note de diarrhée variant de 0 à 2 est attribuée à chaque chevreau :
- 0 = absence de diarrhée,
- 1 = diarrhée pâteuse et
- 2 = diarrhée liquide.

L'excrétion des oocystes ainsi que la note de diarrhée reflètent l'efficacité de la composition testée sur le parasite. Une baisse d'excrétion permet de diminuer la pression d'infestation et qui est généralement accompagnée d'un score se rapprochant de zéro. Elles sont donc intéressantes d'un point de vue épidémiologique.

### (iv) Analyses statistiques

Les résultats sont analysés à l'aide du logiciel Statistica. Des tests de Chi 2, ANOVA, ANOVA à données répétées, coefficient gamma de Goodman et test de Kruskal-Wallis sont utilisés.

### Résultats

### (i) Premier Essai

### Répartition des groupes

Cet essai a été réalisé avec des chevreaux d'origines multiples ce qui présente différents inconvénients : microbisme variable en fonction de l'origine (pasteurellose, colibacillose), chevreaux de race (sans effet sur la cryptosporidiose) et de gabarit différents (effet potentiel sur la capacité à survivre à l'épisode clinique).

Chaque groupe comprenait 11 chevreaux au début de l'expérimentation.

### Validation de l'infection expérimentale

L'infection a été concluante puisque quasiment 100% des chevreaux excrétaient des oocystes 4 jours après l'infection. Les chevreaux de l'élevage 1 ont commencé à excréter des oocystes dès le lendemain de l'infection ce qui témoigne d'une infection dans l'élevage, antérieure à leur arrivée à l'animalerie. Les chevreaux du groupe témoin provenant des élevages 2 et 3 ont commencé à excréter des oocystes 3 jours après l'infection expérimentale. 100% des chevreaux témoins excrétaient des oocystes 4 jours après l'infection (âge moyen des chevreaux : 8 jours) et ce pendant 4 jours.

### Evolution de l'excrétion

L'évolution de l'excrétion est similaire entre le groupe témoin et les groupes traités 2, 3 et 4.

### Note moyenne de diarrhée

Dans le groupe témoin 1, le score moyen de diarrhée a augmenté à partir de l'âge de 7 jours et a commencé à décroître après 15 jours parallèlement à l'excrétion.

Dans les groupes 3 et 4, on a observé une diminution du score moyen de diarrhée sur la période maximale de diarrhée.

La durée moyenne de diarrhée chez les animaux vivants est plus faible dans les groupes 3 et 4 que dans le groupe 1 témoin.

Les chevreaux du groupe 3 ont présenté des signes de diarrhée plus tardivement que les autres groupes et à un niveau moindre.

### Morbidité

La morbidité a été calculée comme le pourcentage de chevreaux présentant de la diarrhée (scores de diarrhée 1 ou 2) par rapport au nombre total de chevreaux présents.

Dans le groupe 1 témoin, plus de 60% des chevreaux ont présenté de la diarrhée entre 8 et 15 jours de façon concomitante à l'augmentation de l'excrétion d'oocystes.

Le taux de morbidité du groupe 4 est moins élevé que dans les autres groupes.

Par ailleurs, le délai dans l'apparition de la diarrhée dans le groupe 3 est plus tardif : alors que plus de 60% des chevreaux des groupes 2 et 4 présentent de la diarrhée dès l'âge de 8 jours, ce pourcentage n'est atteint qu'à l'âge de 11 jours dans le groupe 3.

### Mortalité

21 chevreaux sont morts de façon « naturelle » et 3 euthanasies compassionnelles ont été réalisées. Les chevreaux morts étaient âgés de 5 à 17 jours au moment de la mort. La majorité d'entre eux présentait une excrétion d'oocystes, de la diarrhée et des signes macroscopiques au moment de l'autopsie compatibles avec la cryptosporidiose. Certains des chevreaux ne sont pas morts de la cryptosporidiose dans cet essai.

Aucun effet majeur de l'administration des compositions n'a pu être constaté.

### Conclusion

Dans cet essai, le transport et l'arrivée à l'animalerie ont vraisemblablement été mal supportés par les chevreaux déjà affaiblis par la circulation dans les élevages d'agents pathogènes responsables de maladies pulmonaires et 5 chevreaux sont morts avant la mise en lot. Ceci peut expliquer que les résultats des compositions selon l'invention sont moins spectaculaires que ceux attendus.

Toutefois, la composition A a eu un effet bénéfique sur la diarrhée et la morbidité des chevreaux par rapport à ceux du groupe non traité.

La dose de 5 g par chevreau administrée deux fois par jour a donné les meilleurs résultats.

### (ii) Deuxième Essai

### Répartition des groupes

Ce 2ème essai a été conduit avec des chevreaux d'une seule et même origine, de ce fait très homogène en âge, en poids et sans pathologies intercurrentes. L'extrême sensibilité des chevreaux à l'infection expérimentale confirme l'absence d'infection préalable par les cryptosporidies. Chaque groupe comprenait 11 chevreaux au début de l'essai.

### Validité de l'infection expérimentale

La totalité des chevreaux du groupe témoin 1 a excrété des oocystes de *C. parvum* de façon massive à partir de l'âge de 8 jours (note moyenne d'excrétion : 4,5 sur une échelle de 0 à 5), soit 4 jours après l'infection expérimentale. Le seul chevreau survivant du groupe témoin 1 a excrété des oocystes en continu pendant 8 jours.

### Evolution de l'excrétion

L'évolution de l'excrétion d'oocystes a été comparable dans les groupes 2, 3 et 4 recevant respectivement les compositions B, C et D.

La moyenne des notes d'excrétion la plus élevée dans les groupes 2, 3 et 4 était inférieure à celle du groupe témoin.

L'excrétion moyenne a été plus faible dans le groupe 3 entre 8 et 15 jours que dans le groupe témoin 1 et les groupes 2 et 4.

### Note moyenne de diarrhée

La diarrhée est apparue plus tôt dans les groupes 2, 3 et 4 que dans le groupe 1 témoin, en particulier dans le groupe 3 où plus de 35% des chevreaux étaient diarrhéiques avant 6 jours. L'épisode de diarrhée dû à la cryptosporidiose a ensuite été similaire entre les groupes 2, 3 et 4 et le groupe 1 témoin.

A noter que l'interprétation de la comparaison avec le groupe témoin au-delà de 13 jours a été rendue impossible par la présence d'un seul chevreau survivant dans le groupe témoin.

Le pourcentage de chevreaux diarrhéiques a été légèrement inférieur dans les groupes 2, 3 et 4 par rapport au groupe témoin 1.

La moyenne des notes de diarrhée la plus élevée a été nettement inférieure dans les groupes 2, 3 et 4 (respectivement 1,7, 1,7 et 3,7) par rapport au groupe témoin a (4,5).

La durée de la diarrhée chez les chevreaux vivants a été réduite dans les groupes 2, 3 et 4 par rapport au groupe témoin.

Entre 12 et 18 jours d'âge, les notes de diarrhée sont significativement (p<0,005) plus élevées dans le groupe 1 que dans le groupe 2 ; entre 16 et 17 jours, elles sont plus élevés dans le groupe 1 que dans le groupe 3 ; et entre 17 et 18 jours, elles sont plus élevées dans le groupe 1 que dans le groupe 4.

### Morbidité

La morbidité a été calculée comme le pourcentage de chevreaux présentant de la diarrhée (scores de diarrhée 1 ou 2) par rapport au nombre total de chevreaux présents. Dans le groupe témoin, plus de 80% des chevreaux ont présenté de la diarrhée entre 7 et 12 jours d'âge, de façon concomitante à l'augmentation de l'excrétion d'oocystes.

### Mortalité

14 chevreaux sont morts de façon « naturelle » et 13 euthanasies compassionnelles ont été réalisées. Le pic de mortalité, tous groupes confondus, a été observé à l'âge de 10-11 jours, suite à l'épisode de diarrhée.

La mortalité cumulée dans le groupe 1 a été de 91%, dans le groupe 2 de 36%, dans le groupe 3 de 64% et dans le groupe 4 de 55%.

La mortalité dans le groupe 1 est significativement plus élevée dans le groupe 2.

Si on exclut le chevreau du groupe 4 qui n'est pas mort de la cryptosporidiose, la mortalité est significativement plus importante dans le groupe témoin 1 que dans le groupe 4.

La mortalité dans le groupe 3 est réduite.

A noter que l'âge moyen au moment de la mort est plus élevé dans les groupes traités (13,5 pour le groupe 2 ; 11,7 pour le groupe 3 ; 16,4 pour le groupe 4) que dans le groupe témoin (10,7).

### Conclusion

Les compositions B, C et D selon l'invention ont permis d'améliorer les performances zootechniques, réduire la diarrhée et diminuer la mortalité.

La composition C a également permis de diminuer l'excrétion des oocystes.

### EXEMPLE 5 : TRAITEMENT DE LA CRYPTOSPORIDIOSE INDUITE CHEZ LE SOURICEAU

### (i) Compositions testées

Écorces de levure (Safmannan, France)

### (ii) Test de l'effet des composés sur le développement de C. parvum

Les souriceaux sont inoculés avec 5x10⁵ oocystes de *C. parvum.*

L'administration par voie orale de 500 µg d'écorces de levure, ci-après également dénommées « parois » est réalisée la veille de l'infection par *C. parvum,* ainsi que le lendemain et 4 jours après l'infection, par gavage des souriceaux C57BL/6 âgés de 2 à 3 jours.

Les oocystes présents dans le contenu intestinal, excrétés par voie orale, sont comptés à 6 jours post-inoculation.

Nombre de souriceaux :
- Expérience 1 : n=7-15 souriceaux
- Expérience 2 (n=13 pour chaque lot).

L'analyse statistique a été effectuée en comparant le lot non traité avec chacun des lots (Test non paramétrique Mann Whitney : ** p<0.01)

### Résultats

A J6 post-infection, la charge parasitaire intestinale permet d'évaluer le niveau de protection obtenu par rapport aux animaux infectés mais non traités.

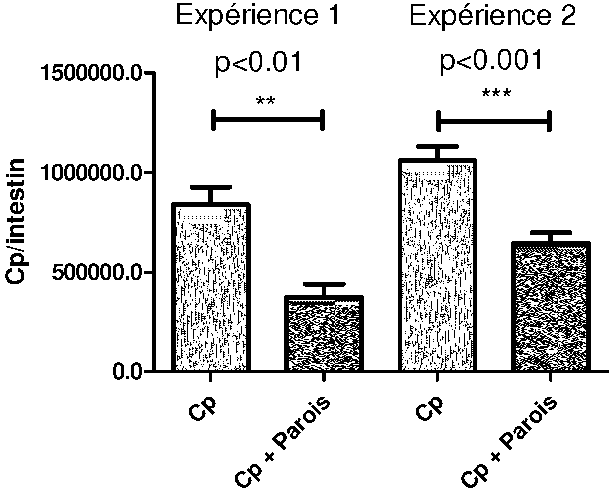

Le graphe représente les résultats de 2 expériences indépendantes. L'administration de parois induit une diminution significative (p<0.01) des oocystes dans les deux expériences.

### Interprétation :

L'administration d'écorces de levures permet une diminution significative de l'excrétion d'oocystes.

## Revendications

1. Composition comprenant :
- au moins un agent de base choisi parmi la chitine, le chitosan, la N-acétyl-glucosamine ou la glucosamine, et
- un agent pour stimuler l'immunité qui est un mélange d'écorces de levure, d'un extrait de levure, de sélénium, et d'une bactérie du genre *Bacillus.*

2. Composition selon la revendication 1, **caractérisée en ce qu'**elle comprend en outre un agent antiparasitaire choisi parmi une huile essentielle, du charbon actif, de l'acide laurique ou leurs combinaisons.

3. Composition selon la revendication 1 ou la revendication 2, **caractérisée en ce qu'**elle comprend en outre un autre agent pour stimuler l'immunité qui est un microorganisme choisi parmi une bactérie du genre *Lactobacillus, Bifidobacterium, Enterococcus, Propionibacterium, Pediococcus* ou *Lactococcus* ou une levure du genre *Saccharomyces* ou *Kluyveromyces,* ou leurs combinaisons.

4. Composition selon la revendication 3, **caractérisée en ce que** le sélénium est sous la forme d'une levure enrichie en sélénium, de sélénium extrait totalement ou partiellement de levure ou leurs combinaisons.

5. Composition selon la revendication 2, **caractérisée en ce que** l'huile essentielle est choisie parmi l'huile essentielle d'ail, l'huile essentielle de citronnelle, l'huile essentielle de cannelle, l'huile essentielle de thym, l'huile essentielle d'origan, l'huile essentielle d'arbre à thé, l'huile essentielle de citron, l'huile essentielle d'eucalyptus ou leurs combinaisons.

6. Composition selon l'une des revendications 1 à 5, **caractérisée en ce que** ladite composition est une composition alimentaire, un complément alimentaire ou une composition pharmaceutique.

7. Chitine, chitosan, N-acétyl-glucosamine, glucosamine ou composition selon l'une quelconque des revendications 1 à 6 pour une utilisation dans la prévention et/ou le traitement d'une parasitose.

8. Chitine, chitosan, N-acétyl-glucosamine, glucosamine ou composition pour une utilisation selon la revendication 7, **caractérisée en ce que** la parasitose est la cryptosporidiose.

## Patentansprüche

1. Zusammensetzung, umfassend:
- mindestens einen Basiswirkstoff, der unter Chitin, Chitosan, N-Acetylglucosamin oder Glucosamin ausgewählt ist, und
- einen Wirkstoff, um die Immunität zu stimulieren, der eine Mischung aus Heferinde, einem Hefeextrakt, Selen und einem Bakterium der Gattung *Bacillus* ist.

2. Zusammensetzung nach Anspruch 1, **dadurch gekennzeichnet, dass** sie ferner einen Antiparasitenwirkstoff umfasst, der ausgewählt ist unter einem ätherischen Öl, Aktivkohle, Laurinsäure oder ihren Kombinationen.

3. Zusammensetzung nach Anspruch 1 oder Anspruch 2, **dadurch gekennzeichnet, dass** sie ferner einen weiteren Wirkstoff, um die Immunität zu stimulieren, umfasst, der ein Mikroorganismus ist, ausgewählt unter dem Bakterium der Gattung *Lactobacillus, Bifidobacterium, Enterococcus, Propionibacterium, Pediococcus* oder *Lactococcus* oder einer Hefe der Gattung *Saccharomyces* oder *Kluyveromyces* oder ihren Kombinationen.

4. Zusammensetzung nach Anspruch 3, **dadurch gekennzeichnet, dass** das Selen in Form einer mit Selen angereicherten Hefe, von Selen, das zur Gänze oder teilweise aus Hefe extrahiert ist, oder ihrer Kombinationen vorhanden ist.

5. Zusammensetzung nach Anspruch 2, **dadurch gekennzeichnet, dass** das ätherische Öl unter ätherischem Knoblauchöl, ätherischem Citronellaöl, ätherischem Zimtöl, ätherischem Thymianöl, ätherischem Oreganoöl, ätherischem Teebaumöl, ätherischem Zitronenöl, ätherischem Eukalyptusöl oder ihren Kombinationen ausgewählt ist.

6. Zusammensetzung nach einem der Ansprüche 1 bis 5, **dadurch gekennzeichnet, dass** die Zusammensetzung eine Nahrungszusammensetzung, eine Nahrungsergänzung oder eine pharmazeutische Zusammensetzung ist.

7. Chitin, Chitosan, N-Acetylglucosamin, Glucosamin oder Zusammensetzung nach einem der Ansprüche 1 bis 6 für eine Verwendung in der Prävention und/oder Behandlung einer Parasitose.

8. Chitin, Chitosan, N-Acetylglucosamin, Glucosamin oder Zusammensetzung für eine Verwendung nach Anspruch 7, **dadurch gekennzeichnet, dass** die Parasitose die Cryptosporidiose ist.

## Claims

1. A composition comprising:
- at least one base agent chosen from chitin, chitosan, N-acetylglucosamine or glucosamine, and
- an agent for stimulating immunity which is a mixture of yeast hulls, a yeast extract, selenium and a bacteria of the *Bacillus* genus.

2. The composition as claimed in claim 1, **characterized in that** it further comprises an antiparasitic agent chosen from an essential oil, active carbon, lauric acid, or combinations thereof.

3. The composition as claimed in claim 1 or 2, **characterized in that** it further comprises another agent for stimulating immunity which is a microorganism chosen from a bacterium of the *Lactobacillus, Bifidobacterium, Enterococcus, Propionibacterium, Pediococcus* or *Lactococcus* genus or a yeast of the *Saccharomyces* or *Kluyveromyces* genus, or combinations thereof.

4. The composition as claimed in claim 3, **characterized in that** the selenium is in the form of a selenium-enriched yeast, selenium totally or partially extracted from yeast, or combinations thereof.

5. The composition as claimed in claim 2, **characterized in that** the essential oil is chosen from garlic essential oil, citronella essential oil, cinnamon essential oil, thyme essential oil, oregano essential oil, tea tree essential oil, lemon essential oil, eucalyptus essential oil, or combinations thereof.

6. The composition according to any one of claims 1 to 5, **characterized in that** said composition is a food composition, a food supplement or a pharmaceutical composition.

7. Chitin, chitosan, N-acetylglucosamine, glucosamine or composition according to any one of claims 1 to 6, for use in the prevention and/or treatment of a parasitosis.

8. Chitin, chitosan, N-acetylglucosamine, glucosamine or composition for use according to claim 7, **characterized in that** the parasitosis is cryptosporidiosis.
